# EUROPÄISCHE PATENTSCHRIFT

(11) **EP 1 248 133 B1**
(45) Veröffentlichungstag und Bekanntmachung des Hinweises auf die Patenterteilung: **09.07.2008**
(21) Anmeldenummer: 02007156.9
(22) Anmeldetag: 29.03.2002
(51) Int. Cl.: G02B 7/00, G02B 21/00

(54) **Mikroskopstativ für ein Operationsmikroskop**
Microscope support for use with a surgical microscope
Pied pour un microscope d'intervention chirurgicale

(30) Priorität: 31.03.2001 DE 10115837
(43) Veröffentlichungstag der Anmeldung: 09.10.2002
(73) Patentinhaber: Leica Microsystems (Schweiz) AG, 9435 Heerbrugg (CH)
(72) Erfinder: Metelski, Andrzej, 8590 Romanshorn (CH)
(74) Vertreter: Rosenich, Paul

(56) Entgegenhaltungen:
- EP-A- 1 067 419
- WO-A-98/53244
- CH-A- 625 057
- DE-C- 967 157
- US-A- 5 366 193

## Beschreibung

In der Chirurgie aber auch in anderen Bereichen der Technik, wie Mikroelektronik, Kriminalistik usw. finden mehr und mehr Operationsmikroskope Anwendung, die infolge ihres hohen Eigengewichts von Stativen getragen werden müssen. Eine Reihe namhafter Hersteller brachte Stative auf den Markt, die in mechanischer und statischer Hinsicht den Anforderungen der Lastaufnahme des Operationsmikroskops gut entsprechen. Die Anmelderin vertreibt z.B. Stative mit der Bezeichnung OHS oder MS1. Ein Beispiel für ein solches Stativ findet sich in der EP-A-628290. Zeiss/Deutschland veröffentlichte ein Stativ z.B. in der EP-476552.
Die CH-A-625 057, Contraves AG, Zürich, offenbart ein verstellbares Stativ für ein optisches Beobachtungsgerät gemäss dem Oberbegriff des Anspruchs 1.

Viele der moderneren Stative verfügen über Parallelogrammträger, um die Last der Operationsmikroskope über möglichst große Distanzen biege- und verwindungsfrei tragen zu können, sodass die Bewegungsfreiheit und der Aktionsradius der Mikroskope möglichst groß sind. Je größer der Aktionsradius, desto größer ist jedoch grundsätzlich die Labilität von Stativen, es sei denn, dass entsprechende konstruktive Maßnahmen gegen Labilität getroffen werden. Je steifer (weniger labil) die Aufbauten, desto mehr neigen sie jedoch zu einem Schwingungsverhalten, dem man ebenso mit konstruktiven Maßnahmen wie Wahl von variierenden Rohrquerschnitten, Werkstoffwahl, Einsatz von Dämpfungselementen usw. begegnet.

Ebenso stellt das Transportgewicht der Stative ein Problem dar, dessen Lösung grundsätzlich in einer Gewichtsreduktion durch hochfeste Materialien liegt. So schuf die Anmelderin z.B. ein Stativ, das wenigstens einen Träger aus einem faserverstärkten Kunststoff einsetzt. Dieses Stativ ist in der erwähnten WO-A-97/20166 beschrieben.

Dabei wurde jedoch erkannt, dass die Gewichtsreduktion alleine u.U. nicht ausreichend ist, sofern dabei die Qualität der Dämpfungseigenschaften der wesentlichen Bauteile nicht ausreichend beachtet wird. Die bloße Gewichtsreduktion führt u.U. zu einem verstärkten, höherfrequenten Schwingungsverhalten des Aufbaus. Verstärkt wird dieses Schwingungsverhalten bei Aufbauten mit gebremsten Armen. Solche Bremsen sind elektromagnetisch, pneumatisch oder auch von Hand zu bedienen und erzeugen eine starre Verbindung zwischen den Bauteilen, sodass Schwingungen von einem Bauteil auf einen anderen übertragen werden und eine für Anwender störende, lange Schwingungszeit erzielen.

In einem anderen Gebiet des Stativbaus, nämlich in der Röntgentechnik, wurde ebenso der Weg der Gewichtsreduktion mittels Faserverbundstoffen und Kunststoff gegangen, wie in der DE-C1-42 14 858 dargelegt ist. Dort wurde ein C-Bogen als Tragteil aus Kunststoff-Schaum geschaffen, der die Gestalt bestimmt, und von einem faserverstärkten Kunststoff umgeben ist, der die Trageigenschaften übernimmt. Soll dieser bekannte Aufbau ein besonders geringes Gewicht aufweisen, so wird gemäß dieser vorveröffentlichten Lehre verlangt, ein Profil in geschlossener Form aus (nur) faserverstärktem Kunststoff herzustellen. Solche Verbundstoffaufbauten haben in sich ein geringes Schwingungsverhalten.

Es gibt bei Stativen für die angegebenen Verwendungen jedoch Gelenke, Drehlager u.dgl, in denen unabhängig von der Qualität der übrigen Bauteile ein Schwingungsverhalten auftreten kann. Eine solche Stelle ist beispielsweise das vertikale Drehlager an einer vertikalen Ständersäule für den oder die horizontalen Tragarme des Stativs. Ausgehend von solchen Lagerstellen, die in der Regel durch Bremsen blockierbar sind, entstehen durch Bewegungen oder Kräfte am Mikroskop auch Torsionskräfte, die wiederum begünstigt Torsionsschwingungen in den auf Torsion belasteten Bauteilen anregen können.

Zur besonderen Schwingungsdämpfung hat die Anmelderin bereits Lösungen angeboten, wie sie z. B. in der WO-A-98/53244 angegeben sind. Dort werden u.a. unter den Aufstellfüßen des Stativfußes dämpfungselastische Schichten angebracht, die in der Schwingungskette vom Mikroskop bis zum Fußboden dämpfend wirken.

Beim kleinsten Lageverändern des Mikroskops kommt es bei diesen bekannten Aufbauten zu einer Schwingungserregung, die jedoch, nachdem sie das Stativ durchlaufen hat, an den Aufstellfüßen gedämpft wird und so nur noch abgeschwächt zurückgeworfen wird.

Ebenso wurden Dämpfungsplatten vorgestellt, die zwischen Stativbauteilen eingesetzt werden, so z.B. Dämpfungsschuhe beim Übergang von einem Trägerrohr zu einer Trägerrohr-Fassung oderDämpfungsptatten zwischen zwei Flanschen zweier benachbarter Trägerrohre oder einem Rohr und einem Sockel.

Der Vorteil solcher Dämpfungselemente im Bereich des Hauptkörpers des Stativs ist, dass sie helfen, die Schwingungen schon auf ihrem Weg vom Mikroskop zum Boden zu dämpfen, damit sie nicht erst das ganze Stativ durchlaufen müssen. Die Wirksamkeit dieser bekannten Dämpfungszwischenlagen ist dabei die Dämpfungswirkung, die bei Kompression dieser Dämpfungselemente auftritt, das ist also z.B., wenn das Rohr in seinem Schuh in Axialrichtung des Rohres oder in einer Richtung senkrecht darauf schwingt (Kippschwingung) bzw. wenn die Aufstellfüße durch ein Schwingen der Ständersäule in einer Vertikalebene auf Druckbelastungsschwankungen belastet werden.

Torsionsschwingungen wurden bisher dadurch zu dämpfen versucht, indem man die Trägerrohre besonders ausgebildet hat. So hat man z.B. Aluminium-Kunststoff-Verbundrohre oder carbonfaserverstärkte Kunststoffrohre geschaffen, bei denen durch eine bestimmte Wahl der Faserlagen Torsionen im Rohr bekämpft wurden. Das so aufgebaute OHS der Anmelderin hat aber nicht nur aufgrund der guten Wahl der Träger ein geringes Torsionsverhalten, sondern auch wegen des waagenförmigen Aufbaus um die Drehachse in der Ständersäule. Bei diesem bekannten Aufbau liegt der Schwerpunkt der bewegten Tragarme und Balancierarme direkt über oder in unmittelbarer Nähe der Ständersäule. Andere Stative, die den Schwerpunkt der bewegten Tragarme deutlich seitlich der Ständersäule haben, vergrößern das Torsionsschwingungsverhalten; vor allem, wenn das Stativ über der Drehachse gebremst ist. Schon das Lösen oder schließen der Bremse, oder kleinste Bewegungen am Mikroskop können Torsionsschwingungen erzeugen.

Torsionsschwingungen (häufig Horizontalschwingungen) sind bei der Mikroskopie insbesondere deshalb wesentlich schädlicher als Vertikalschwingungen, weil bei einer Vertikalschwingung durch die grundsätzlich vorhandene Tiefenschärfe eine geringe Schwingung nicht wahrgenommen wird. Horizontale Schwingungen führen jedoch zu starken Beeinträchtigungen bei der Beobachtung durch das Mikroskop.

Aufgabe der vorliegenden Erfindung ist es, Lösungen zu finden, die das Schwingungsverhalten des Stativs verbessern, d.h. die Schwingung unterbinden oder allfällige Schwingungen optimal dämpfen, ohne dabei auf eine präzise Positionstreue zu verzichten. Dabei sollen insbesondere niederfrequente Torsionsschwingungen, z.B. im Bereich von z.B. 0-10 Hz, bekämpft werden. Die neuen Maßnahmen sollen Torsionsschwingungen wirksam bekämpfen und gegebenenfalls in Kombination mit bekannten Schwingungsdämpfungsmaßnahmen einsetzbar sein.

Der Fachmann weiß, dass solche Aufgaben schwer lösbar sind und dass die Anwendung mathematisch-physikalischer Hilfsmittel und Theorien häufig nicht die erwarteten Resultate bringt. Andererseits sind aber schon geringfügige Verbesserungen erstrebenswert, da diese den Komfort des Anwenders steigern und demzufolge die Operationssicherheit anheben.

Gelöst wird diese Aufgabe erfindungsgemäß durch die im Anspruch 1 angegebenen Merkmale.

Die Erfindung bietet somit für die zwingend vorhandenen Bauteile an einem Stativ für ein Operationsmikroskop besonders geeignete und abgestimmte Dämpfungselemente mit geringerem Gewicht und verbessertem Schwingungsverhalten. Die Anforderungen an Stativträgerteile können hinsichtlich deren Schwingungsverhalten etwas reduziert werden, was hier zu Kostensenkungen führen kann.

Die Erfindung sieht eine Bremse vor und dabei kann das Dämpfungselement unmittelbar oder mittelbar an einem drehkraftübertragenden Teil der Bremse angebracht sein, insbesondere mit der Bremse in Serie geschaltet sein.

Die Erfindung sieht vor, dass das Dämpfungselement im gebremsten Zustand unbelastet, also frei von Spannungen, auch von Axialkräften gehalten ist.

Des Weiteren sind in einer bevorzugten Ausgestaltung der Erfindung Schwingungswegbegrenzer vorgesehen.

Weitere spezielle Ausbildungen und Varianten dazu sind in den Patentansprüchen beschrieben bzw. unter Schutz gestellt.

Die Eigenschaften des bevorzugten Materials liegen bei etwa folgenden Parametern:
Statischer Elastizitätsmodul 0,2 - 3 N/mm²,
dynamischer Elastizitätsmodul 0,5 - 4 N/mm²
mechanischer Verlustfaktor 0,1 - 0,2
Eigenfrequenz des Materials über 5 Hz
jeweils gemessen in Anlehnung an DIN 53513.

Als bevorzugtes Material wird beispielhaft Sylomer® M12, Sylomer® M25 P14 oder Sylomer® P12, Sylomer® P25 P15 oder insbesondere Sylodamp® HD-010-11, HD300/1, HD-030-11, HD-050-21, HD-100-11, HD-150-12, HD-300-10 oder 12, bevorzugt jedoch HD-300-1 für den dynamischen Lastbereich von 0-0,3 N/mm² gewählt.

Der Verlustfaktor gemäß ISO 10846-2 sollte bei 8 Hz bevorzugt mehr als 0,1, insbesondere mehr als 0,2 betragen bei einer Reißdehnung nach DIN 53455-6.4 von über 100%, vorzugsweise von über 200% und insbesondere von ca. 300%.
Lieferbar sind solche Materialien unter der Bezeichnung SYLODAMP® von Getzner Werkstoffe GmbH Bürs/Österreich.

Bei Bedarf können dämpfende Materialien auch kombiniert werden. Ebenso liegen im Rahmen der Erfindung Varianten mit bestimmter Formgebung an den Dämpfungsmaterialien. Z.B. können Ausnehmungen, wie Sacklöcher o.dgl. vorgesehen sein, um das Dämpfungsverhalten weiter zu beeinflussen.

### Figurenbesehreibung

Die Figuren werden zusammenhängend beschrieben. Die Figurenbeschreibung und die Bezugszeichenliste bilden eine Einheit, die sich durch die übrigen Teile der Beschreibung und Ansprüche im Sinne einer vollständigen Offenbarung gegenseitig ergänzen. Gleiche Bezugszeichen bedeuten gleiche Bauteile. Gleiche Bezugszeichen mit unterschiedlichen Indizes bedeuten ähnliche, funktionsgleiche Bauteile. Die Figuren sind nur beispielhaft und nicht zwingend proportional richtig dargestellt.

Es zeigen dabei:
- Fig.1: eine Schrägansicht eines erfindungsgemäßen Stativ-Drehlagers beim Übergang zwischen der Ständersäule und einem Tragarm;
- Fig.2: einen vertikalen Schnitt durch den Aufbau gem. Fig.1;

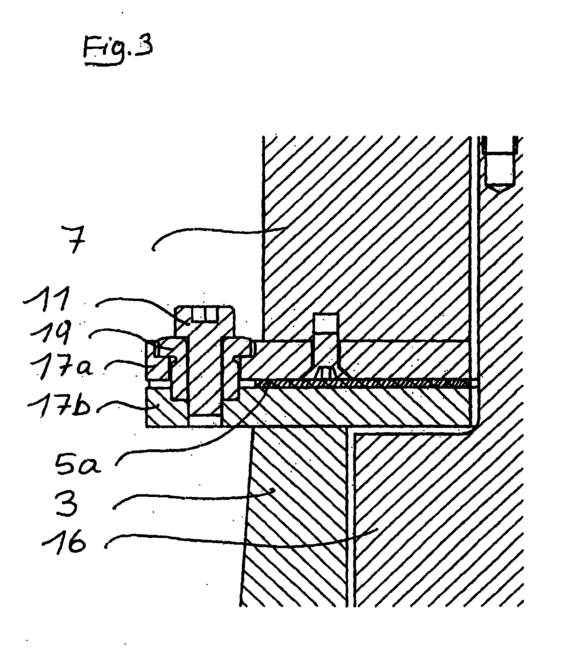

- Fig.3: ein vergrößertes Detail aus Fig.2;

Die Figuren werden übergreifend beschrieben. Gleiche Bezugszeichen bedeuten gleiche Gegenstände; gleiche Bezugszeichen mit unterschiedlichen Indizes bedeuten Bauteile mit gleichen oder ähnlichen Aufgaben aber unterschiedlicher Bauart. Die Bezugszeichenliste ist integrierender Bestandteil der Figurenbeschreibung.

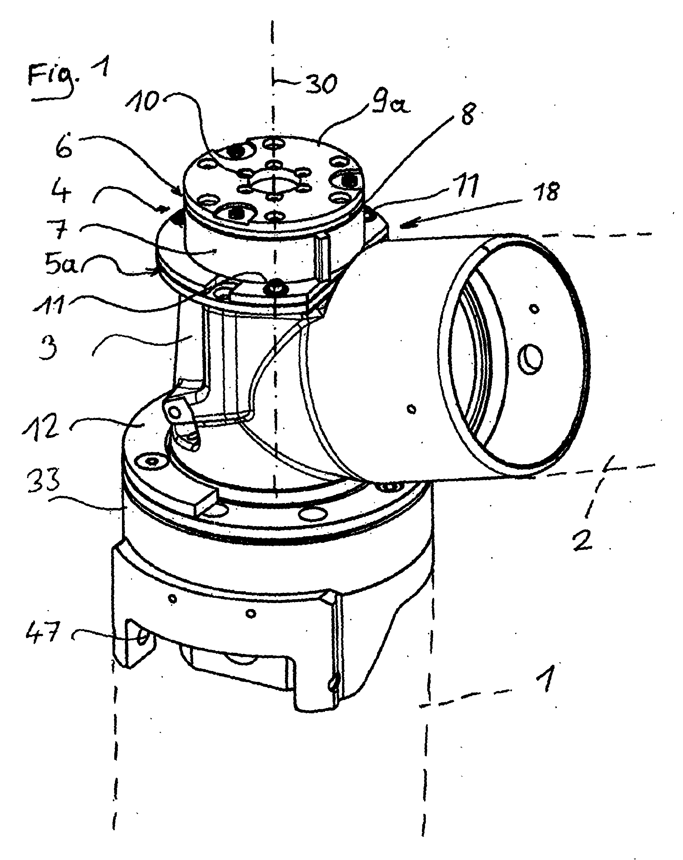

Fig.1 zeigt einen realisierten Aufbau des erfindungsgemäßen Stativs im Ausschnitt. Dieser Aufbau schließt unmittelbar an die am gleichen Tag eingereichte Patentanmeldung DE 101 23 166 an, die sich mit einem anderen Detail möglicher Stativausstattungen auseinandersetzt.

An einer nur angedeuteten Ständersäule 1 ist eine - vorzugsweise gemäß der DE 101 23 166 ins Lot stellbare - Lagerhülse 33 vorgesehen, die einen Tragkörper 3 trägt. Mit dem Tragkörper 3 ist ein nur angedeuteter Tragarm 2 verbunden, wie er beispielsweise in Fig.12 der Patentanmeldung DE 101 23 166 mit 11c bezeichnet ist. Der Tragarm 2 ist um eine Drehachse 30 drehbar, damit er seine Last (ein Mikroskop) in verschiedene Raumlagen bringen kann. Um eine gewählte Raumlage zu fixieren, ist eine Bremse 4 vorgesehen, die den Tragarm 2 im gebremsten Zustand relativ zur Ständersäule 1 festhält. Ist die eingebremste Position erreicht, können - auch schon kleine - seitliche Wechselkräfte an der Last (am Mikroskop) zu einer Schwingungsanregung führen, die zu einem Hin- und Herschwingen der Last führen. Dabei werden Torsionskräfte in der Bremse 4, im Stativständer 1 und im Tragarm selbst (als Biegekräfte) wirksam. Dieses Hin- und Herschwingen möglichst zu unterdrücken oder zu kompensieren ist die vorderste Aufgabe der Erfindung. Bewerkstelligt wird dies beim Aufbau gem. Fig.1 durch ein Torsionsdämpfungselement 5a, das zwischen Bremsangriffsfläche 6 und Tragkörper 3 angeordnet ist.

Die Bremse 4 umfasst im Wesentlichen einen Bremskörper 7 und einen Anker 8 sowie einen Ankerflansch 9a. Der Bremskörper 7 ist mit dem Tragkörper 3 und der Ankerflansch 9a bzw. der Anker 8 ist mit der Ständersäule 1 kraftschlüssig verbunden. Die Verbindung zum Tragkörper 3 ist durch Schrauben 11 bewerkstelligt, während die Verbindung zur Ständersäule 1 über die Schrauben 10 erfolgt.

An der Ständersäule 1 festgelegt ist auch ein Schwenkbegrenzer 12, der im Zusammenhang mit einem bestimmt ausgebildeten Stativfuß und einem zum Gewichtsausgleich dienenden Gerätekasten (vgl. Fig. 12) der Patentanmeldung DE 101 23 166 den Erfindungseffekt der Patentanmeldung PCT/EP98/03614 ergibt und insofern auch unter Schutz gestellt ist.

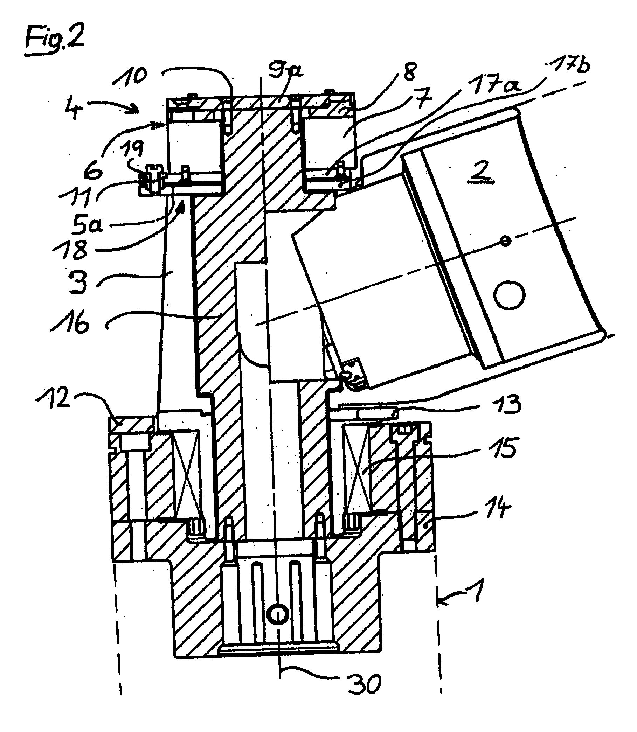

Der Schwenkbegrenzer 12 kooperiert dabei mit einem Anschlag 13 am Tragkörper 3 (Fig.2).

Wie besser aus Fig.2 ersichtlich, umfasst die Ständersäule 1 einen Lagerblock 14, der ein Lager 15 trägt, in dem der Tragkörper 3 gelagert ist. Konzentrisch innerhalb des Tragkörpers befindet sich ein Ankerbock 16, der mit dem Lagerblock 14 starr verbunden ist und an seinem oberen Ende über den Ankerflansch 9a den Anker 8 trägt. Die Achse 30 der Ständersäule 1 bildet somit die Drehachse für den Tragkörper 3 und damit für den Tragarm 2.

Im Rahmen der Erfindung liegen selbstverständlich auch beliebige andere Aufbauten, bei denen keine oder eine andere Ständersäule vorgesehen ist, bzw. wo die Funktion der Ständersäule durch andere Bauteile übernommen wird, wie beispielsweise bei Dekkenstativen die Deckensäule oder bei Wandstativen die Wandhalterung oder bei Stativen mit mehreren Tragarmen einer dieser.

Die Funktionsweise der - in diesem Fall elektromagnetischen - Bremse 4 und des erfindungsgemäßen Aufbaus ist wie folgt: Im nichterregten Zustand der Bremse 4 bzw. des Bremskörpers 7 liegt - wie in Fig.2 dargestellt - der Anker 8 an der Bremsangriffsfläche 6 am Bremskörper 7 an. Daher ist zwischen dem Tragkörper 3 und dem Ankerbock 16 - und damit der Ständersäule 1 - keine Rotation möglich. Die Bremskraft wird somit von der Ständersäule 1 über den Lagerblock 14 in den Ankerbock 16, von dort über den Ankerflansch 9a auf den Anker 8 und den Bremskörper 7 übertragen, um von diesem über einen Dämpfungsflansch 18 auf den Tragkörper 3 und damit auf den Tragarm 2 übertragen zu werden.

Der Dämpfungsflansch 18 umfasst einen oberen und einen unteren Flansch 17a, b, zwischen dem das Dämpfungselement 5a eingelegt oder eingeklebt ist. Der obere und der untere Flansch sind mittels Distanzhülsen 19 getrennt, die einerseits eine gewisse Vorspannung zwischen den beiden Flanschen ermöglichen, andererseits - bedingt durch eine entsprechende Langloch- oder Lochgrößenausbildung aber auch eine relative Verdrehmöglichkeit zueinander um die Achse 30 bieten.

Die Distanzhülsen 19 verhindern zudem, dass bei geöffneter Bremse das Torsionsdämpfungselement 5a auf Zug belastet wird. Das schont - sollte das Torsionsdämpfungselement wie bevorzugt an den Flanschen 17 angeklebt sein - die Klebeverbindung.

Der Anker 8 selbst ist wie nicht näher dargestellt, aber bei solchen Bremsen üblich, federbelastet.

Die Verdrehmöglichkeit um die Distanzhülsen 19 erzeugt ein Spiel, das eine geringfügige Schwenkbarkeit des Tragarms 2 auch bei angezogener Bremse 4 erlaubt. Gegen diese Schwenkbarkeit wirkt nun mit seinen Torsionsrückstellkräften das Torsionsdämpfungselement 5a. Bei der bevorzugten Ausbildung bewirken diese Rückstellkräfte ein annähernd 100%-iges Rückstellen eines im Toleranzbereich bewegten Tragarms 2. Die spezielle Ausbildung bzw. Materialwahl beim Torsionsdämpfungselement 5a führt zu den schwingungsdämpfenden Eigenschaften des Aufbaus.

### Bezugszeichenliste

- 1: Ständersäule
- 2: Tragarm
- 3: Tragkörper
- 4: Bremse
- 5: a Torsionsdämpfungselement
- 6: Bremsangriffsfläche
- 7: Bremskörper
- 8: Anker
- 9: a Ankerflansch
- 10: Schrauben
- 11: Schrauben
- 12: Schwenkbegrenzer
- 13: Anschlag
- 14: Lagerblock
- 15: Lager
- 16: Ankerbock
- 17: a, b Flansch
- 18: Dämpfungsflansch
- 19: Distanzhülsen
- 30: Achse
- 33: Lagerhülse
- 47: Schwenkachse siehe DE 101 23 166 (für die vorliegende Erfindung nicht wesentlich.

## Patentansprüche

1. Mikroskopstativ für Operationsmikroskope mit einer vertikalen Ständersäule (1) mit einer Achse (30) und einem horizontalen Tragarm (2), der um die Achse (30) der vertikalen Ständersäule (1) horizontal schwenkbar-angeordnet ist, und einer Schwingungsdämpfung, wobei der Tragarm (2) mit einem Tragkörper (3) verbunden ist, der in einem Drehlager (15) der Ständersäule (1) gelagert ist, welches die horizontale Schwenkung des Tragkörpers (3) mit dem daran befestigten Tragarm (2) relativ zu der Ständersäule (1) um die Achse (30) ermöglicht, und wobei eine feststellbare bzw. anziehbare Bremse (4) an der vertikalen Ständersäule (1) angeordnet ist, mithilfe derer der Tragarm (2) im gebremsten Zustand relativ zur Ständersäule (1) fixiert wird, und wobei die Schwingungsdämpfung als Torsionsdämpfungselement (5) gegen Torsionskräfte aufgrund horizontaler Schwenkbewegungen des horizontalen Tragarms (2) gegenüber der vertikalen Ständersäule (1) um die Achse (30) bei angezogener Bremse (4) ausgebildet ist, **dadurch gekennzeichnet, dass** das Torsionsdämpfungselement (5) von Axialkräften freigehalten ist und rein auf Scherung beansprucht wird.

2. Stativ nach Anspruch 1, **dadurch gekennzeichnet, dass** die Rückstellkräfte des Torsionsdämpfungselements (5) so groß sind, dass sie eine Rückstellung eines gegen die Wirkrichtung des Torsionsdämpfungselements verschobenen Stativbauteils zu wenigstens 90% vorzugsweise zu wenigstens 99% bewirken, wobei vorzugsweise das Torsionsämpfungselement (5) aus einem zelligen Kunststoffschaum aufgebaut ist und vorzugsweise wenigstens einen der folgenden Parameter aufweist: Statischer Elastizitätsmodul 0,2 - 3 N/mm², dynamischer Elastizitätsmodul 0,5 - 4 N/mm², mechanischer Verlustfaktor 0,1 - 0,2 der Eigenfrequenz des Materials über 5 Hz, jeweils gemessen in Anlehnung an DIN 53513;
der Verlustfaktor bei 8 Hz gemäss nach ISO 10846-2 sollte bevorzugt mehr als 0,1, insbesondere mehr als 0,2 sein;
Reissdehnung nach DIN 53455-6.4 von über 100%, vorzugsweise von über 200% und insbesondere von ca. 300%.

3. Stativ nach Anspruch 1, **dadurch gekennzeichnet, dass** das Torsionsdämpfungselement (5a) als wenigstens eine Dämpfungsplatte, vorzugsweise als Dämpfungsscheibe ausgebildet ist und dass das Dämpfungselement (5a) als Dämpfungsplatte zwischen zwei relativ zu einander verdrehbaren Bauteilen eingeklebt ist.

4. Stativ mit wenigstens einer Bremse nach einem der vorhergehenden Ansprüche, **dadurch gekennzeichnet, dass** das Torsionsdämpfungselement (5) unmittelbar oder mittelbar an einem drehkraftübertragenden Teil der Bremse (4) angebracht ist, insbesondere mit der Bremse (4) in Serie geschaltet ist.

5. Stativ nach einem der vorhergehenden Ansprüche, **dadurch gekennzeichnet, dass** Schwingungswegbegrenzer (12, 13) bereitgestellt sind.

## Claims

1. Microscope stand for surgical microscopes, having a vertical post (1) with an axis (30), and a horizontal support arm (2) which is arranged in a fashion capable of pivoting horizontally about the axis (30) of the vertical post (1), and having a vibration damper, the support arm (2) being connected to a support body (3) which is mounted in a rotary bearing (15) of the post (1), which enables the support body (3) with the support arm (2) fastened thereon to pivot horizontally about the axis (30) relative to the post (1), and there being arranged on the vertical post (1) a brake (4) which can be locked or applied and with the aid of which the support arm (2) is fixed relative to the post (1) in the braked state, and the vibration damper being designed as a torsional damping element (5) countering torsional forces on the basis of horizontal pivoting movements of the horizontal support arm (2) with respect to the vertical post (1) about the axis (30) with the brake (4) applied, **characterized in that** the torsional damping element (5) is kept free of axial forces and is subjected solely to shear.

2. Stand according to Claim 1, **characterized in that** the restoring forces of the torsional damping element (5) are so great that they effect a return of a stand component, displaced against the effective direction of the torsional damping element, of at least 90%, preferably at least 99%, the torsional damping element (5) preferably being constructed of a cellular plastic foam and preferably having at least one of the following parameters: static modulus of elasticity 0.2 - 3 N/mm², dynamic modulus of elasticity 0.5 - 4 N/mm², mechanical loss factor 0.1 - 0.2; natural frequency of the material greater than 5 Hz, measured in each case on the basis of DIN 53513;
the loss factor at 8 Hz in accordance with ISO 10846 - 2 preferably being greater than 0.1, in particular greater than 0.2;
breaking extension according to DIN 53455-6.4 of greater than 100%, preferably of greater than 200%; and in particular of approximately 300%.

3. Stand according to Claim 1, **characterized in that** the torsional damping element (5a) is designed as at least one damping plate, preferably as a damping disc, and **in that** the damping element (5a) is bonded in as a damping plate between two mutually rotatable components.

4. Stand having at least one brake according to one of the preceding claims, **characterized in that** the torsional damping element (5) is fitted directly or indirectly on a part of the brake (4) which transmits rotary forces, in particular is connected in series with the brake (4) -

5. Stand according to one of the preceding claims, **characterized in that** vibration travel limiters (12, 13) are provided.

## Revendications

1. Socle pour microscope chirurgical, qui présente une colonne verticale (1) de socle dotée d'un axe (30) et d'un bras horizontal de support (2) agencé de manière à pouvoir pivoter horizontalement autour de l'axe (30) de la colonne verticale (1) de socle, ainsi qu'un amortisseur de vibrations, le bras de support (2) étant relié à un corps de support (3) monté dans un palier de rotation (15) de la colonne (1) du socle, lequel palier permet au corps de support (3) et au bras de support (2) qui y est fixé de pivoter par rapport à la colonne (1) du socle autour de l'axe (30), un frein (4) apte à être fixé et actionné étant disposé sur la colonne verticale (1) de socle et permettant lorsqu'il est serré d'immobiliser le bras de support (2) par rapport à la colonne (1) du socle, l'amortisseur de vibrations étant configuré comme élément (5) d'amortissement de torsion qui, lorsque le frein (4) est serré, amortit les forces de torsion qui résultent des déplacements de pivotement horizontal du bras horizontal de support (2) par rapport à la colonne verticale (1) du socle autour de l'axe (30),
**caractérisé en ce que**
l'élément (5) d'amortissement de torsion ne subit pas l'action des forces axiales et est sollicité purement par frottement.

2. Socle selon la revendication 1, **caractérisé en ce que** les forces de rappel de l'élément (5) d'amortissement de torsion sont suffisamment élevées pour entrainer un rappel d'au moins 90 % et de préférence d'au moins 99 % d'un composant du socle déplacé dans le sens opposé à l'action de l'élément d'amortissement de torsion, l'élément (5) d'amortissement de torsion étant constitué d'une mousse cellulaire de matière synthétique et présente de préférence au moins l'un des paramètres suivants, mesurés selon la norme DIN 53 513 : module d'élasticité statique de 0,2 à 3 N/mm², module d'élasticité dynamique de 0,5 à 4 N/mm², facteur de perte mécanique de 0,1 à 0,2 lorsque 1a fréquence propre du matériau est supérieure à 5 Hz, le facteur de perte à 8 Hz, mesuré selon la norme ISO 10846-2, devant de préférence être supérieur à 0, 1 et en particulier à 0,2, et allongement à la rupture selon la norme DIN 53 455-6.4 supérieur à 100 %, de préférence supérieur à 200 % et en particulier environ 300 %.

3. Socle selon la revendication 1, **caractérisé en ce que** l'élément (5a) d'amortissement de torsion est configuré sous la forme d'au moins une plaque d'amortissement, de préférence d'un disque d'amortissement, et **en ce que** l'élément (5a) d'amortissement configuré en plaque d'amortissement est collé entre deux composants aptes à tourner l'un par rapport à l'autre.

4. Socle doté d'au moins un frein selon l'une des revendications précédentes, **caractérisé en ce que** l'élément (5) d'amortissement de torsion est installé directement ou indirectement sur une partie du frein (4) qui transmet des forces de rotation et est en particulier raccordé en série au frein (4).

5. Socle selon l'une des revendications précédentes, **caractérisé en ce qu'**il présente des limiteurs (12, 13) de déplacements de vibration.
